(19) **Europäisches Patentamt / European Patent Office / Office européen des brevets**

(11) **EP 4 190 222 A1**

(12) # EUROPEAN PATENT APPLICATION

(43) Date of publication:
**07.06.2023 Bulletin 2023/23**

(21) Application number: **21211769.1**

(22) Date of filing: **01.12.2021**

(51) International Patent Classification (IPC):
**A61B 1/00** (2006.01)

(52) Cooperative Patent Classification (CPC):
**A61B 1/000095; A61B 1/000094; A61B 1/00096; A61B 1/00186; G02B 23/2423; A61B 1/00101**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **PAULUSSEN, Elvira Johanna Maria**
**Eindhoven (NL)**

• **LAI, Marco**
**Eindhoven (NL)**
• **HENDRIKS, Bernardus Hendrikus Wilhelmus**
**Eindhoven (NL)**
• **REICH, Christian**
**Eindhoven (NL)**
• **COMPEN, Hendrikus Antonius Cornelus Maria**
**Eindhoven (NL)**
• **SHAN, Caifeng**
**Eindhoven (NL)**

(74) Representative: **Philips Intellectual Property & Standards**
**High Tech Campus 52**
**5656 AG Eindhoven (NL)**

(54) **ENDOSCOPIC IMAGING**

(57) An endoscope system is for generating an image of tissue to be examined. The system is adapted to deliver radiation with a first polarization to the tissue, and a return polarization control system is provided in the path between the tissue and the receiver. It has an electrically controllable adjuster with an array of adjuster pixels which are independently controllable to implement a local polarization control in respect of local regions of the image. In this way, the image can be enhanced in a pixelated way to give the best possible imaging of tissue beneath the surface.

$$S_{//} \cdot R_{//}$$
$$S_{//} \cdot R_{\perp}$$
$$S_{\perp} \cdot R_{//}$$
$$S_{\perp} \cdot R_{\perp}$$

$$S_{//} \text{ or } S_{\perp}$$

FIG. 15

EP 4 190 222 A1

**Description**

FIELD OF THE INVENTION

This invention relates to endoscopic imaging.

BACKGROUND OF THE INVENTION

Endoscopes are widely used to provide imaging of internal body tissue. To analyze tumor tissue, it is desired to use infrared wavelengths that can penetrate deeper into human tissue than visible light. This can enable tumor margins to be identified of tiny tumors that lie underneath healthy tissue.
For this purpose, so-called hyperspectral imaging (HSI) cameras are known for analyzing a wider range of colors, in particular using a wavelength range that extends beyond the visible range.
For tumors located in deeper organs, like brain tumors, endoscopy is used to visualize the organs via a lens system (ocular) or to image the tissue on a screen. For minimally invasive neurosurgical procedures, endoscopic images are used for navigating inside the skull base to reach the tumor, as in endo-nasal skull-base surgery. If an endoscope is connected to a hyperspectral imaging (HSI) camera, tissue classification, e.g. tumors detection, can be enabled.

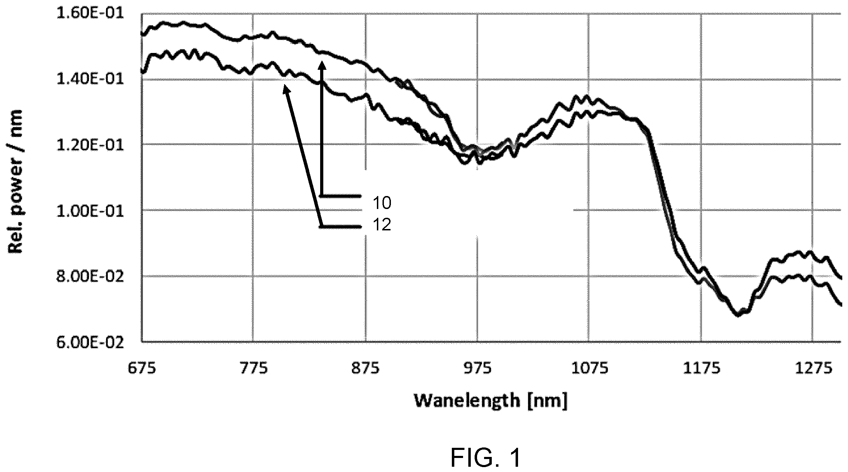

Figure 1 shows an infrared spectral response of normal brain tissue (plot 10) and tumorous brain tissue (plot 12) analyzed in an optical simulation model that is similar to an endoscopic hyperspectral imaging system.
Table 1 shows reported volume fractions (%) for the different components water, blood and fat for normal and tumor tissue.

Table 1

| Volume fraction [%] | f water | f blood | f_fat |
|---|---|---|---|
| Brain Tissue (white, grey matter) | 72 | 2.7 | 25.3 |
| Brain tumor tissue | 53 | 5.7 | 41.2 |

From the study of S. Nazeer ("Fluorescence spectroscopy to discriminate neoplastic human brain lesions: a study using the spectral intensity ratio and multivariate linear discriminant analysis, Laser Physics 2014:24(2):025602) on differences between adjacent normal and tumor tissues (Table 1), the absorbance is derived from the volume fractions of components for healthy and tumor brain tissue by Formula [1]:

Formula [1]

$$\mu_a = f_{blood} \cdot CHGb_{blood} \cdot \mu_{a, oxy} + f_{blood} \cdot (1 - CHGb_{blood}) \cdot \mu_{a, deoxy} + f_{water} \cdot \mu_{a, water} + f_{fat} \cdot \mu_{a, fat}$$

The reduced scattering coefficient $\mu'_s$ is derived by Formula [2] with parameters found by Sandell and Zhu (A review of in-vivo optical properties of human tissue and its impact on PDT, J. Biophotonics, 2011 Nov 4(11-12); 773-787):

Formula [2]

$$\mu'_s = a \cdot \left( \frac{\lambda}{500 \, nm} \right)^{-b}$$

$$\mu_s = \frac{\mu'_s}{(1-g)}$$

Table 2 shows the parameters for specifying the reduced scattering coefficient.

Table 2

|  | Average | Brain Tissue | Tumor Tissue |
|---|---|---|---|
| a | 24.2 | 40.8 | 33.6 |
| b | 1.611 | 3.089 | 1.712 |
| a' | 27.4 | 40.8 | 37.3 |
| f | 0.315 | 0 | 0.72 |
| b_mie | 1.087 | 3.088 | 0 |

The different optical properties for $\mu_a$ and $\mu_s$ for brain normal and tumorous tissue result into the spectral response curve in Figure 1.

From Figure 1, it is clear that in the infrared range from 675 to 1275 nm and from the depth information of the tissue, tumorous tissue can be distinguished from non-tumorous tissue.

The typical illumination optics of a rigid endoscope contains a waveguide system, for example of one or more optical fibers, that guides light from an external light source to the distal end of the endoscope. Typically, the efficiency of such a system with fibers is low (~20%) mainly due to etendue. More advanced endoscopes use more efficient LED illumination. The LED can be connected to a light pipe that transports the heat away from the distal end, avoiding the temperature of the distal end from rising. The LED may be a point source at the distal end of the endoscope or a ring-shaped LED or multiple LEDs can be applied at the distal end of the endoscope.

A problem with a conventional endoscope imaging using visible light is that tumorous tissue can hardly be visualized. By making use of wavelengths in the infrared region as explained above, tumorous tissue can be distinguished from vital tissue by means of the spectral properties.

Compared to visible light, infrared rays penetrate to deeper layers of the tissue, resulting in imaged spectral response information of deeper layers.

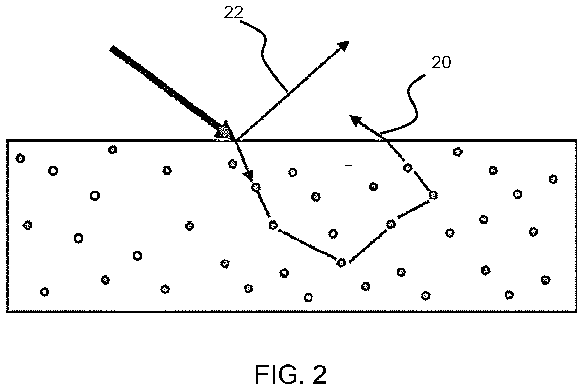

Figure 2 shows scattered radiation 20 which can be detected. However, an issue when analyzing the spectral response is that there always exists a specular reflectance component 22 of the air-to-tissue interface, resulting in an offset in the spectral reflectance curve of Figure 1. The back scattering component scattered back by scattering particles in the tissue can be overshadowed by the specular reflectance component at the air-tissue boundary.

It is known to suppress specular reflections by using polarizers, for example as disclosed in WO 2020/077237. However, using polarization results in a loss of light intensity and can thus reduce image brightness.

SUMMARY OF THE INVENTION

The invention is defined by the claims.

According to examples in accordance with an aspect of the invention, there is provided an endoscope system for generating an image of tissue to be examined, comprising:

a radiation source for generation imaging radiation;
an optical waveguide for delivering the imaging radiation to the tissue, wherein the system is adapted to deliver radiation with a first polarization to the tissue;
a receiver for receiving radiation reflected or scattered by the tissue; and
a return polarization control system in the path between the tissue and the receiver, comprising an electrically controlled adjuster having an array of adjuster pixels which are independently controllable to implement a local polarization control in respect of local regions of the image.

This system uses two polarization control functions, one for the delivered radiation and one for the returned radiation. The two polarization functions may for example be crossed (either because they are fixed in a crossed state or they may be adjustable into a crossed state), thereby to block specularly reflected light from the tissue surface. In this way, the contrast of the imaging of tissue below the surface is improved.

However, rather than blocking all specularly reflected light in this way, a pixelated adjuster is provided. The adjuster is pixelated so that selected pixels can result in a crossed polarization relative to the first polarization (or equivalently, the polarization can be controlled to be uncrossed relative to the first polarization). The pixels of the adjuster correspond to pixels of the eventual image and hence also correspond to particular regions of the tissue being imaged. In this way, the light loss associated with the use of the crossed polarization functions (which will also block some light scattered from the deeper tissue) can be avoided for chosen areas of the image. Thus, if specular reflection is not an issue for parts of the image, polarization control can allow more imaging radiation to pass. Thus, a higher contrast image can be obtained.

The first and second polarization functions are preferably linear polarization functions.

The radiation source preferably generates visible and infrared radiation. The radiation source and receiver for example together define a hyperspectral imaging camera sensor.

The system for example comprises optics which create at least one image plane, and wherein the electrically controllable adjuster is located at the at least one image plane. Thus, the endoscope image is present within the optical system and the pixelated adjuster is located where the image is formed, so that different pixels of the adjuster correspond to different regions of the image.

The system for example further comprises a control system for controlling the pixels of the electrically controllable adjuster in dependence on the local level of surface specular reflection. In this way, when specular reflection needs to be suppressed, the adjuster can allow crossed polarizations to filter out the specular reflection, or when specular reflection does not need to be suppressed, the adjuster can prevent the specular reflections being filtered out and hence also prevent that light with the same polarization state as the reflected components being filtered out.

In one implementation, the return polarization system comprises:

a return polarizer; and
an electrically controllable adjuster for adjusting a polarization direction for local regions of the image.

The implementation thus uses two (fixed) polarizers and a pixelated rotator between them. These three components allow good local contrast.

The first polarization and the polarization direction of the return polarizer may be crossed (so that radiation will only pass if there is a polarization rotation between them). However, they could also be parallel, so that the crossed polarization blocking function only takes place when a polarization rotation is provided.

The electrically controllable adjuster for example comprises a liquid-crystal based pixelated LC polarization rotator. This is used to provide an electrically controllable polarization rotation.

In one example, the system comprises a first polarizer in the path between the radiation source and the tissue. This enables use of a non-polarized radiation source. This first polarizer is not needed if the radiation source has a polarization conversion system.

The electrically controllable adjuster may then be between the first polarizer and the return polarizer. The first polarizer for example comprises part of a cover placed over the distal end of the optical waveguide. In another possible example, the electrically controllable adjuster may be between the radiation source and the first polarizer.

In one example, the polarizers have a fixed angular orientation. However, a manual adjuster may be provided for adjusting the polarization direction of the first and second polarizers relative to each other. The manual adjuster for example comprises a first rotary control for adjusting an angular orientation of the first polarizer and a second rotary control for adjusting an angular orientation of the second polarizer. The first polarizer may be at a distal end of the optical waveguide and the second polarizer may be at a proximal end of the optical waveguide.

The polarizers for example comprise wire grid polarizers. The rotation is about the propagation direction of the radiation.

In another example, the electrically controllable adjuster comprises a pixelated polarizer. In this case, fewer components are needed, hence giving good local contrast and local brightness. It enables the first polarization to pass or to be blocked, thus selectively blocking the specular reflections or allowing them to pass.

Pixelated wire-grid polarizers are for example known. The polarizers with different polarization directions could then be aligned with camera pixels. The selection of the camera pixels used to form an image then enables different polarization images to be obtained.

The optical waveguide may be a polarization maintaining optical fiber.

The radiation source may then be controllable to generate radiation with a selected polarization state, e.g. p-polarization or s-polarization, hence avoiding the need for the first polarizer as outlined above. This can be used to generate an image based on a crossed return polarizer (compared to the selected polarization state of the radiation source) and an image based on a parallel return polarizer (compared to the selected polarization state of the radiation source). In this way, the system can select between an optimum surface image or an optimum image for deeper tissue.

The system for example comprises a controller for controlling the radiation source to enable image acquisition alternately in the two polarization states of the radiation source. The two image types may be combined using image and data processing, for example to obtain an image representing a random polarization state of the source.

The invention also provides an endoscope imaging method for generating an image of tissue to be examined, comprising:

generating imaging radiation;

delivering the imaging radiation with a first polarization to the tissue;
receiving radiation reflected or scattered by the tissue to be examined after passing through a return polarization control system; and
electrically controlling an adjuster of the return polarization control system, having an array of adjuster pixels, by independently controlling the pixels to implement a local polarization control in respect of local regions of the image.

The method for example further comprising controlling the pixels of the electrically controllable adjuster in dependence on the local level of surface specular reflection.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Figure 1 shows an infrared spectral response of normal brain tissue and tumorous brain tissue;
Figure 2 shows scattered radiation which can be detected from beneath a tissue surface;
Figure 3 shows the optical waveguide of an endoscope and a crossed polarization system is added to the distal end of the optical waveguide;
Figure 4 shows the first polarizer with the second polarizer removed;
Figure 5 shows the two polarizers;
Figure 6 shows an exploded view of the components of Figure 3;
Figure 7 shows the endoscope set-up with a radiation source and a receiver;
Figure 8 shows an endoscopic image of a brain slice taken with a HSI sensor, taken without crossed polarized light;
Figure 9 shows an equivalent image to Figure 8 but using crossed polarizers;
Figure 10 shows an endoscope with a switchable polarizer;
Figure 11 shows an alternative to the design of Figure 10, with the active polarization rotator at the proximal end;
Figure 12 shows an example of a relay lens system;
Figure 13 shows an alternative optical system;
Figure 14 shows the steps of a method for controlling a pixelated adjuster; and
Figure 15 shows the use of two LCD rotators

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an endoscope system for generating an image of tissue to be examined. The system is adapted to deliver radiation with a first polarization to the tissue, and a return polarization control system is provided in the path between the tissue and the receiver. It has an electrically controllable adjuster with an array of adjuster pixels which are independently controllable to implement a local polarization control in respect of local regions of the image. In this way, the image can be enhanced (by blocking specular reflections) in a pixelated way to give the best possible imaging of tissue beneath the surface.

The invention combines the use of cross-polarization to prevent specular reflection components from reaching the receiver of the endoscope imaging system, with pixel-level control of a polarization function so that different parts of an image can be treated differently.

The ways in which the cross-polarization function may be implemented will first be discussed.

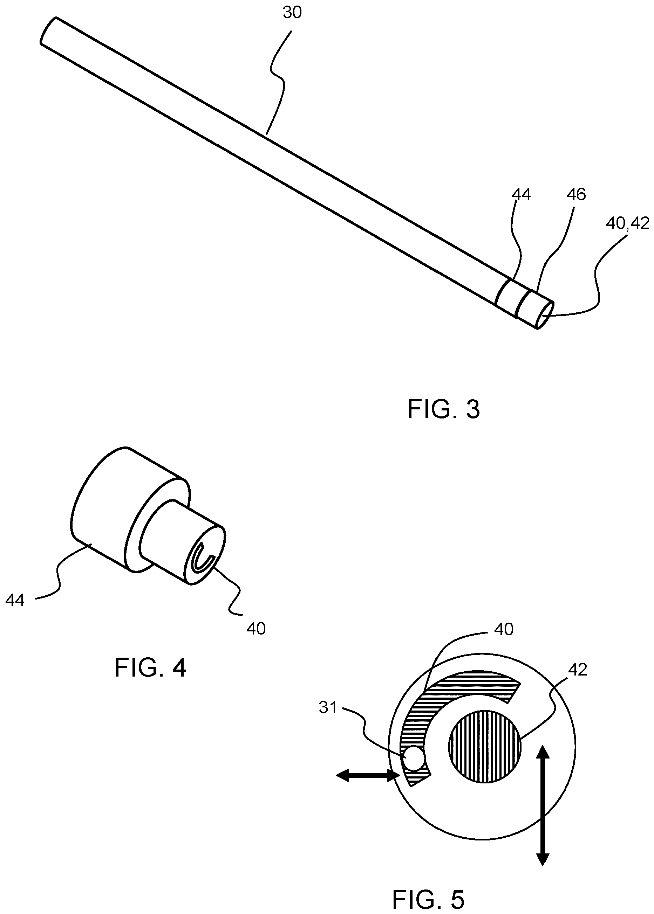

Figure 3 shows the optical waveguide 30 of an endoscope. It comprises an illumination waveguide. For the return of the image, a relay lens system may be used, and it provides the returned image to an image sensor or to a viewer. The proximal end (or a junction near the proximal end) of the optical waveguide connects to a radiation source and the distal end is for delivering imaging radiation for internal imaging of a patient.

In this example, a crossed polarization system is added to the distal end of the optical waveguide 30.

The specular reflection contains no depth-information of the tissue so this component can be removed using crossed polarizers. Thus, there is a first linear polarizer 40 in the path from the radiation source to the tissue. The first polarizer 40 is for example placed in front of the tissue, and is in the radiation path exiting the illumination waveguide 30. A second linear polarizer 42 may be considered to comprise an analyzer, and it is in the reflected path from the tissue back to a receiver at the proximal end of the optical waveguide 30. Thus, it is at the entrance to the relay lens system.

The waveguide system of a typical rigid optical system contains a cone shaped optics and a fiber bundle in which single glass fibers are embedded into a light absorbing adhesive. Because of the set up with a fiber bundle, the polarization state is not maintained and the fiber can be a non-polarization maintaining fiber (non-PMF) fiber. Because the illumination waveguide thus changes the polarization direction, the polarizer position in this example is at the distal end of the endoscope and hence in front of the illumination light path. The analyzer position can be at the distal end as well, in front of the imaging light path as shown.

Normally, the relay lens system of an endoscope does not change the polarization direction. Consequently, an alternative position for the analyzer is in front of the image sensor at the proximal end of the endoscope.

There are thus various solutions for integrating a crossed polarizer function in the endoscope.

In the example shown, the first polarizer 40 is placed in front of the illumination waveguide end and it can be rotated over a 360° angle by rotation knob 44. Thus, it can be rotated relative to the (unknown) polarization direction present at the distal end of the optical waveguide.

Figure 4 shows the first polarizer 40 with the second polarizer removed.

The second polarizer 42, in front of the imaging light path, has a 90° crossed orientation with respect to the first polarizer 40. Both polarizers may be wire grid polarizers that function in the infrared range, where tumorous tissue can be analyzed via a hyperspectral imaging camera sensor. A separate rotation knob 46 is shown in Figure 3 for the second polarizer 42.

Thus, in this example, the first and second polarizers can be rotated independently, and the 90° crossing angle is not fixed. However, the two polarizers may instead be rotated as a unit while maintaining the orthogonal relationship. The rotation enables the overall polarizer module to be aligned relative to polarization of the light delivered by the illumination waveguide.

Figure 5 shows the two polarizers. Polarizer 40 is in the path of light from the illumination waveguide, the end of which is shown as 31. When the first polarizer is rotated, the angle of the wire grid (at the location 31 where the illumination waveguide terminates) changes. Similarly, rotation of the second polarizer changes the wire grid orientation where the reflected radiation is delivered to the relay lens system.

The two polarizers are in this example fixed by means of a socket cap. The first polarizer can for example be rotated over 360° in a plane perpendicular to the imaging surface (XY in Figures 3 and 4) by rotation around the Z axis.

The illumination waveguide is for example a fiber bundle. The polarizer has an arcuate shape so that when it is rotated, it remains over the end 31 of the waveguide. The polarizer may have other shapes, such as a half-moon shape. Other shapes are of course possible, in this example, the return path is along the center and hence the second polarizer is centrally positioned.

In use, the first polarizer 40 is first aligned for maximum light throughput of the illumination waveguide. Subsequently, the second polarizer 42 of the analyzer is rotated to 90° with respect to the first polarizer until maximum contrast is observed in the image.

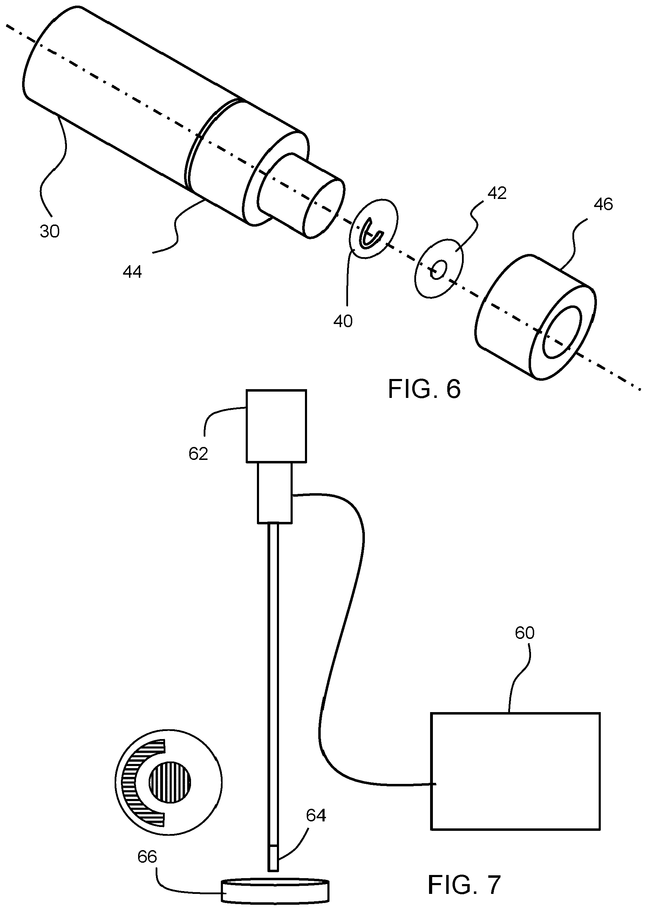

Figure 6 shows an exploded view of the components.

Figure 7 shows the endoscope set-up with a radiation source 60 such as a halogen illuminator for generating imaging radiation, and a receiver 62 in the form of a HSI camera for receiving radiation reflected or scattered by the tissue. The distal end of the endoscope is fitted with the polarization unit 64 explained above and a sample 66 to be imaged is shown. The polarization unit 64 may instead be integrated inside the endoscope to reduce the insertion diameter.

The polarization unit 64 comprises the first polarizer 40 which means radiation is delivered with a first polarization to the tissue sample 66 and the second, return, polarizer in the path between the tissue and the receiver, crossed with the first polarizer.

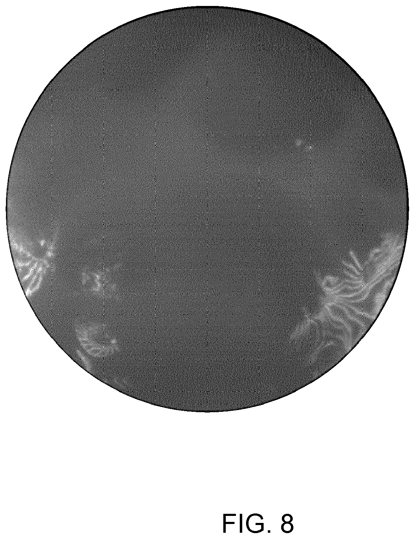

Figure 8 shows an endoscopic image of a brain slice taken with a HSI sensor, which contains grey and white brain tissue. In Figure 8 the image is taken without crossed polarized light, and the issue of a specular component can be seen on the sides of the image, as well as the low contrast between the grey and white matter.

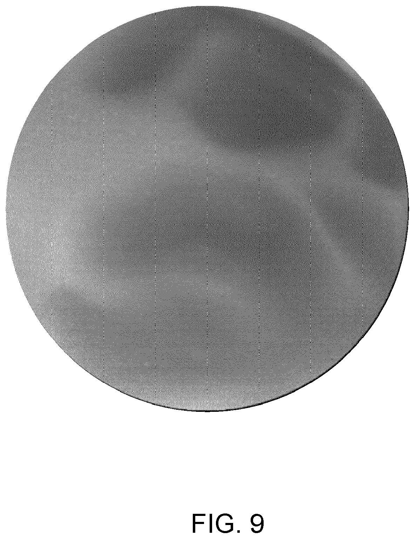

Figure 9 shows an equivalent image to Figure 8 but using the crossed polarizers, with the polarization unit rotated until the maximum contrast is reached. The contrast is improved by the use of the crossed polarizers, reducing the specular component and improving also the contrast between grey and white matter.

The wavelength range analyzed by the HSI spectral camera is for example 675 to 950 nm, or 900nm to 1700nm for an InGaAs detector sensor.

In use of an endoscope during surgery, it may not need to be in contact with tissue (i.e. micro surgery) or it may make some minimal contact with tissue (i.e. minimally invasive surgery or keyhole surgery). However, in both options the distal end is in close proximity with the tissue or even touches the tissue. For this reason, it may be desired to use a prophylactic cover around the endoscope with an integrated polarizer enclosing and protecting the distal end of the waveguide.

The analyzer can also be integrated in such a cover or, to have more space for the polarizer and to prevent any misalignment at the narrow distal end, the analyzer can be positioned at the proximal end.

The arrangement described above is fixed, so that the specular reflections are suppressed. However, it may also be desirable to obtain an image (or part of the image) without suppression of the specular reflections. This may be achieved by providing a polarization rotation between the first and second polarizers. Polarization rotation can be implemented electrically (instead of manually as is possible with the two knobs shown above).

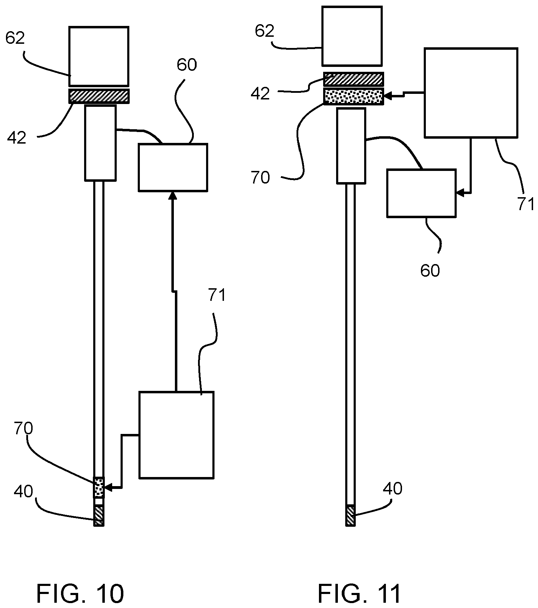

Figure 10 shows an endoscope with a switchable polarizer 70. In the example of Figure 10, an active polarization rotator component is used that rotates the polarization direction by adapting a voltage. It is integrated in the distal end of the endoscope. The first polarizer 40 is at the distal end, and in this example, the second polarizer 42 is at the proximal end before the receiver 62. A controller 71 controls the switchable polarizer 70. As explained below, it may also control the radiation source 60.

The active polarization rotator can be a liquid crystal (LC) component or another electro-optical (EO) component.

Figure 11 shows an alternative with the active polarization rotator 70 at the proximal end, because the polarization direction is not changed by the relay lens system. A controller 71 controls the polarization rotator 70.. As explained below, it may also control the radiation source 60.

The polarizer 40 in the distal end can be integrated in front of a protection window or can replace (partly) the protection window for example by providing a coated.

The invention is based on the use of an electrically controllable adjuster, such as an optical polarization rotation system, but which in particular has an array of adjuster pixels which are independently controllable to implement a local polarization rotation in respect of local regions of the image. Thus, the adjuster is a pixelated version of the polarizer or rotator 70.

The number of pixels desired will depend on the application. For example, a minimum size may be 2x2 pixels, but it may for example be of the same order of magnitude of the pixels of the receiver sensor. $100 \times 100$ pixels or more is for example possible. The electrically controllable adjuster is in the path between the radiation source and the return polarizer.

Specular reflectance occurs when a surface is smooth instead of diffuse and the reflected light has the same polarization as the incident light.

However, the light that is diffusively reflected can also have the same polarization direction as the incoming light. When the surface is smooth and flat, the direct (specular) reflectance is visible at the image as glare as explained above.

By placing a pixelated adjuster, such as an LC component, at an imaging position within the relay lens system, the specular reflectance can be reduced by rotating the polarization direction only locally, in particular only at the regions where specular reflectance occurs.

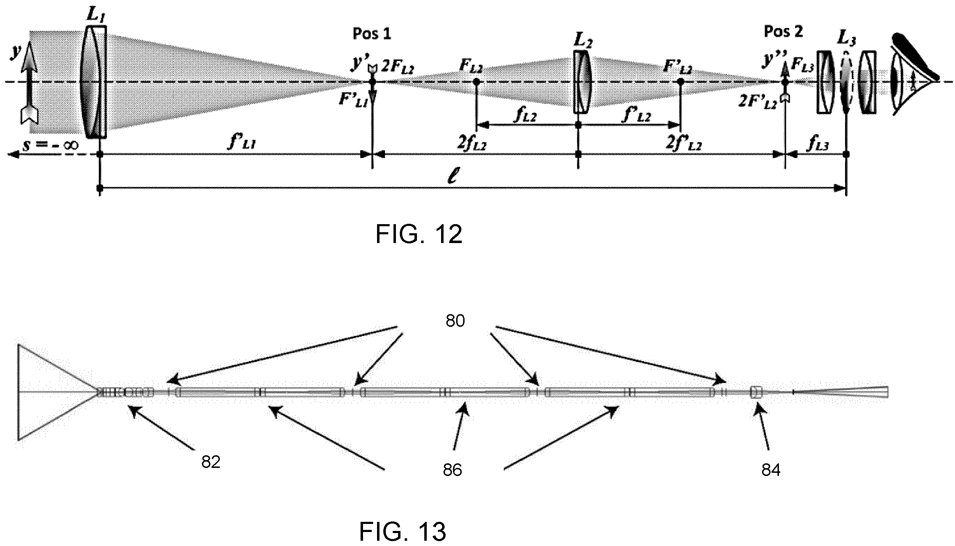

Figure 12 shows an example of a relay lens system. There are two focal points along the lens relay system, giving rise to two image positions Pos1 and Pos2 in the relay lens system.

Both of these positions are suitable for placing an LC component that can rotate the polarization direction.

Figure 13 shows the system with four locations 80 at which images are formed; two in the center, one at the end where the objective system 82 is provided and one image at the eyepiece 84 that fits into the endoscope. The optical relay system has relay sections 86.

The advantage of a pixelated rotation of polarization is that parts of the image which have already high contrast and depth information (because there is little or no specular reflectance component) can be allowed to maintain their light level. In the crossed polarization set-up, the analyzer blocks part of the light that is back scattered by the tissue beneath the surface, when it happens to have the same polarization as the light that is specularly reflected. As a result, at positions where specular reflectance occurs, the contrast and depth information is enhanced locally. The arrangement thus enables some parts of the image to be corrected for specular reflectance, whereas other parts remain uncorrected, which already have good contrast and depth information.

For example, if a yellowish tumor (with low contrast) is surrounded by more fibrous connective tissue (with high contrast), it may be desirable to have the surrounding tissue beneath or above the tumor also visible in high contrast, to give depth perception, but the tumor itself highlighted at the surface.

A liquid-crystal based pixelated LC polarization rotator may be used, as is employed in liquid crystal display (LCD) screens, enabling precisely controlled realignment of liquid crystal molecules between different ordered molecular configurations under the action of an applied electric field. This is achieved with little power consumption and at low operating voltages.

The pixelated rotator is for example placed at a position of the endoscope where an image is formed. It is for example placed at an image plane between the radiation source (e.g. the first polarizer if there is one) and the return polarizer. In this case, the return polarizer is most practically placed at the proximal end. The pixelated rotator is most practically placed just in front of the return polarizer, close to the receiver sensor (camera) image plane. Per pixel, either the horizontal or vertical polarization state passes through.

If the polarization state is controllable, for example using an adjustable first polarizer (not needing to be pixelated), the specular component can be distinguished from reflected light that comes from deeper tissue and can be suppressed per pixel.

The examples above make use of a first polarizer (or a polarized radiation source), a pixelated polarization rotator, and a second polarizer. However, the pixelated polarization rotator and the second polarizer may be replaced by a pixelated polarizer.

The pixelated polarizer, and the combination of a polarization rotator and a second polarizer, are both examples of a return polarization control system, which is in the path between the tissue and the receiver. The electrically controllable adjuster may thus be a polarizer (i.e. a component allowing a selected polarization to pass) or a polarization rotator.

When a pixelated polarizer is used, it may again be placed at an image plane, or, more practically, just in front of the camera at the proximal end. Again by offering different states of polarization to the tissue, the specular component can be distinguished from reflected light that comes from deeper tissue.

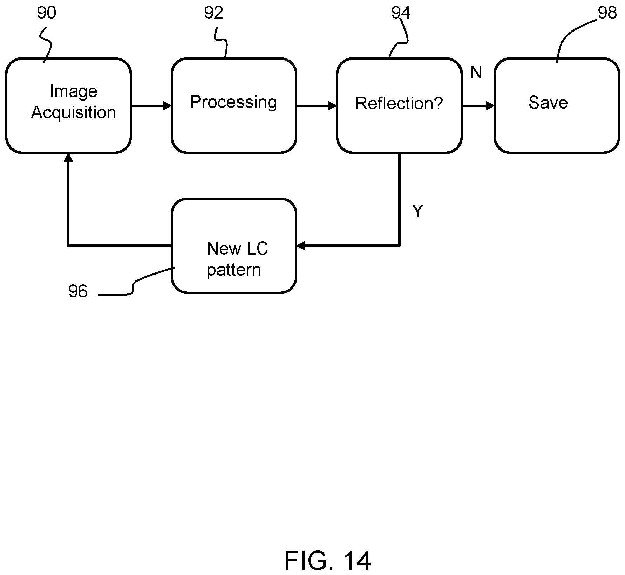

Figure 14 shows the steps of a method for controlling the pixelated adjuster.

The image is acquired by the receiver in step 90. This may be achieved an HSI system, but the invention can equally be applied to an RGB image. The image is digitized in step 92 (and other image processing may be performed). In step 94 it is determined if parts of the image include undesired surface reflections. Any suitable image analysis may be performed to determine if there are specular reflections that may desirably be suppressed. If so, a new activation pattern for the adjuster is obtained in step 96 and the process returns to a new image acquisition. Thus, the process iteratively makes adjustments until reflections have been cancelled in the parts of the image where they have arisen. The best image is saved in step 98.

Thus, the polarization control is adapted per pixel. This may be a binary function (e.g. 90 degree rotation or no rotation) or there may be a finer control of the polarization rotation. The advantage of a pixelated correction is that only the regions where specular reflectance occurs are processed accordingly and other regions remain untreated and keep the original amount of backscattered light. Consequently, the optimal amount of light throughput combined with optimal image quality is obtained.

The example above is based on an unknown polarization entering the first polarizer. However, the first polarizer may be omitted if the incident polarization can be controlled. This is possible if there is controlled polarization by the radiation source.

A polarization-maintaining fiber can be used in endoscopes for wavelengths in the infrared range. In a polarization maintaining fiber, the polarization of linearly-polarized light waves launched into the fiber is maintained during propagation. In such a case, a polarizer can be used just behind the light source (i.e. near the proximal end instead of the distal end- typically the radiation source is at the side near the proximal end such as shown in Figure 7) or a polarization conversion system can be used to convert the light into one polarization state (with an efficiency around 85% and with a 50-75% increase in etendue).

The analyzer (second polarizer) may be placed at the distal end by a compact and thin add-on unit in front of the imaging channel or at the proximal end in front of the receiver (e.g. CCD camera).

A further option is to adapt the radiation source such way that it produces light in two polarization states (p-polarization and s-polarization). This may be achieved by means of an active rotator (LCD or digital micromirror device). This control of the radiation source may be implemented by the controller 71.

With a high frame rate (e.g. 20 frames per second) alternating picture acquisition may be controlled to take place in the p- and s- polarization states. In this way, the two-polarizer system (with the second polarizer of the analyzer at the distal end or the proximal end) is used to record a time frame with parallel polarizations in one frame and with crossed polarizations in the other frame. The optimum image (surface image or deeper tissue image) can be obtained with suitable data processing by combining each of the states. The random state is the sum of the polarization images:

$$I_{random} = I_{parallel} + I_{crossed}$$

Hence:

$$I_{random} = S_{//} \cdot R_{//} + S_{//} \cdot R_{\perp} + S_{\perp} \cdot R_{//} + S_{\perp} \cdot R_{\perp}$$

Wherein:

S// is the source intensity in the parallel polarization state;

R// is the intensity after tissue reflection (after the analyzer in the parallel orientation);

S⊥ is the source intensity in the crossed polarization state;

R⊥ is the intensity after tissue reflection (after the analyzer in the crossed orientation).

Using a liquid crystal rotator to enable the polarization direction of radiation entering the endoscope and another liquid crystal rotator to control the polarization of light allowed to reach the receiver, all four p- and s- combinations can be imaged.

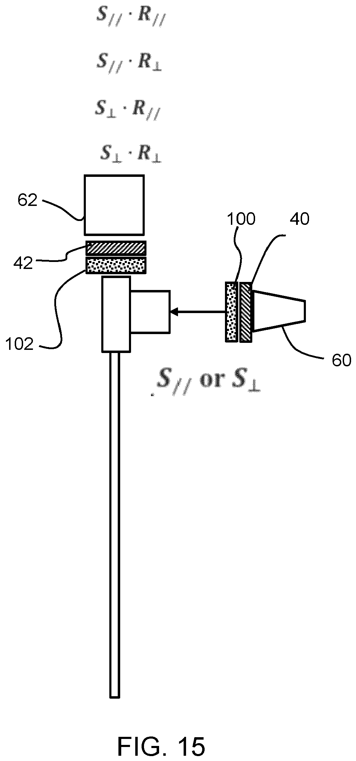

This option is shown in Figure 15.

The radiation source 60 has a first polarizer 40 followed by a first active polarization rotator 100. The receiver 62 has a second active polarization rotator 102 followed by the second polarizer 42.

There are four combinations of polarization states as defined above.

With a fixed second polarizer 42 (i.e. analyzer) at the proximal end (hence without the second polarization rotator 102, only two of the four combinations of polarization states can be imaged.

For p-polarization and s-polarization at a limited angle of incidence, the surface (Fresnel) reflectance is almost equal, so this should not be an issue for endoscopic imaging.

With an external light source at large angle of incidence, there is an increased advantage of taking four polarization states. For this purpose an active rotator (LC component) is integrated both at the source and at the proximal end of the endoscope as shown.

Each of the polarization states of the source S// and S⊥ are controlled in the source by the rotator 100 and each of the polarization states after tissue reflection are controlled by the second polarization rotator 102 at the analyzer. The second polarizer is the pixelated adjuster, since it is at a location where an image is formed.

The result of combining polarization images is the same as the approach explained above, but an iterative process is no longer needed.

The radiation source may be a laser source, LED source, gas discharge lamp or xenon source. Alternatively, combinations of sources are possible.

The polarizer or polarizers are preferably, but not limited to, a wide wavelength range wire grid polarizer. Known wire grid polarizers for example contains an array of thin metal wires (lines) closely spaced, on top of a transparent substrate. It works as an absorptive polarizer and only passes through light oscillating perpendicular to the wires. The transparency is important for the imaging function of the system. Because the wire grid polarizer has a wide wavelength range (visible to infrared) and because of this transparency, it is appropriate for use in the infrared endoscopic imaging system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

**Claims**

1. An endoscope system for generating an image of tissue to be examined, comprising:

   a radiation source (60) for generation imaging radiation;
   an optical waveguide (30) for delivering the imaging radiation to the tissue, wherein the system is adapted to deliver radiation with a first polarization to the tissue;
   a receiver (62) for receiving radiation reflected or scattered by the tissue; and
   a return polarization control system (40,42,70) in the path between the tissue and the receiver, comprising an electrically controllable adjuster (70) having an array of adjuster pixels which are independently controllable to implement a local polarization control in respect of local regions of the image.

2. The system of claim 1, wherein the radiation source (60) generates visible and infrared radiation.

3. The system of claim 1 or 2, comprising optics which create at least one image plane (80), and wherein the electrically controllable adjuster (70) is located at the at least one image plane.

4. The system of any one of claims 1 to 3, further comprising a control system for controlling the pixels of the electrically controllable adjuster (70) in dependence on the local level of surface specular reflection.

5. The system of any one of claims 1 to 4, wherein the return polarization system comprises:

   a return polarizer (42); and
   the electrically controllable adjuster (70) for adjusting a polarization direction for local regions of the image.

6. The system of claim 5, comprising a first polarizer (40) in the path between the radiation source and the tissue.

7. The system of claim 6, wherein the electrically controllable adjuster (70) is:

   between the radiation source (60) and the first polarizer (40); or
   between the first polarizer (40) and the return polarizer (42).

8. The system of claim 6 or 7, comprising a manual adjuster for adjusting the polarization direction of the first and return polarizers (40,42) relative to each other.

9. The system of any one of claims 6 to 8, wherein the first polarizer (40) is at a distal end of the optical waveguide and the return polarizer (42) is at a proximal end of the optical waveguide.

10. The system of any one of claims 1 to 4, wherein the electrically controllable adjuster comprises a pixelated polarizer.

11. The system of any one of claims 1 to 10, wherein the optical waveguide (30) is a polarization maintaining optical fiber.

12. The system of claim 11, wherein the radiation source (60) is controllable to generate radiation with a selected one of two polarization states, wherein the system further comprises a controller (71) for controlling the radiation source to perform image acquisition alternately in the two polarization states.

13. An endoscope imaging method for generating an image of tissue to be examined, comprising:

   generating imaging radiation;
   delivering the imaging radiation with a first polarization to the tissue;
   receiving radiation reflected or scattered by the tissue to be examined after passing through a return polarization control system (40,42,70); and
   electrically controlling an adjuster (70) of the return polarization control system, having an array of adjuster pixels, by independently controlling the pixels to implement a local polarization control in respect of local regions of the image.

14. The method of claim 13, further comprising controlling the pixels of the electrically controllable adjuster (70) in dependence on the local level of surface specular reflection.

FIG. 1

FIG. 2

30

44    46

40,42

FIG. 3

44

40

FIG. 4

40

42

31

FIG. 5

30

44

40

42

46

FIG. 6

62

60

64

66

FIG. 7

FIG. 8

FIG. 9

FIG. 10

FIG. 11

FIG. 12

FIG. 13

90

Image
Acquisition

92

Processing

94

Reflection?

N

98

Save

Y

New LC
pattern

96

FIG. 14

$$S_{//} \cdot R_{//}$$

$$S_{//} \cdot R_{\perp}$$

$$S_{\perp} \cdot R_{//}$$

$$S_{\perp} \cdot R_{\perp}$$

$$S_{//} \text{ or } S_{\perp}$$

FIG. 15

Europäisches
Patentamt

European
Patent Office

Office européen
des brevets

# EUROPEAN SEARCH REPORT

Application Number

EP 21 21 1769

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/271061 A1 (FUKAZAWA TAKANORI [JP] ET AL) 2 September 2021 (2021-09-02) | 1,2,5-7, 9,12,13 | INV. A61B1/00 |
| Y | * paragraph [0077] – paragraph [0333]; figures 1-20 * | 4,10,14 | |
| X | WO 2017/090229 A1 (CANON KK [JP]) 1 June 2017 (2017-06-01) * paragraph [0012] – paragraph [0069]; figures 1-5 * | 1,3,5,8, 11-13 | |
| Y | WO 2018/207569 A1 (SONY CORP [JP]) 15 November 2018 (2018-11-15) * paragraph [0020] * | 4,14 | |
| Y | US 2005/243314 A1 (CHINNOCK RANDAL B [US]) 3 November 2005 (2005-11-03) * paragraphs [0045] – [0051]; figures 1-10 * | 10 | |
| | | | **TECHNICAL FIELDS SEARCHED (IPC)** A61B |

The present search report has been drawn up for all claims

| Place of search | Date of completion of the search | Examiner |
|---|---|---|
| Munich | 4 May 2022 | Mäki-Mantila, M |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
................................................
& : member of the same patent family, corresponding
document

EPO FORM 1503 03.82 (P04C01)

**EP 4 190 222 A1**

## ANNEX TO THE EUROPEAN SEARCH REPORT
## ON EUROPEAN PATENT APPLICATION NO.

EP 21 21 1769

This annex lists the patent family members relating to the patent documents cited in the above-mentioned European search report.
The members are as contained in the European Patent Office EDP file on
The European Patent Office is in no way liable for these particulars which are merely given for the purpose of information.

04-05-2022

| Patent document cited in search report | | Publication date | Patent family member(s) | | Publication date |
|---|---|---|---|---|---|
| US 2021271061 | A1 | 02-09-2021 | JP WO2019159583 | A1 | 04-02-2021 |
| | | | US 2021271061 | A1 | 02-09-2021 |
| | | | WO 2019159583 | A1 | 22-08-2019 |
| WO 2017090229 | A1 | 01-06-2017 | JP 2017098831 | A | 01-06-2017 |
| | | | WO 2017090229 | A1 | 01-06-2017 |
| WO 2018207569 | A1 | 15-11-2018 | US 2020154988 | A1 | 21-05-2020 |
| | | | WO 2018207569 | A1 | 15-11-2018 |
| US 2005243314 | A1 | 03-11-2005 | NONE | | |

## REFERENCES CITED IN THE DESCRIPTION

### Patent documents cited in the description

- WO 2020077237 A

### Non-patent literature cited in the description

- **S. NAZEER.** Fluorescence spectroscopy to discriminate neoplastic human brain lesions: a study using the spectral intensity ratio and multivariate linear discriminant analysis. *Laser Physics,* 2014, vol. 24 (2), 025602

- **SANDELL ; ZHU.** A review of in-vivo optical properties of human tissue and its impact on PDT. *J. Biophotonics,* November 2011, vol. 4 (11-12), 773-787